Europäisches Patentamt

European Patent Office

Office européen des brevets

(19)

(11) Numéro de publication: **0 245 129 B1**

(12) **FASCICULE DE BREVET EUROPEEN**

(45) Date de publication de fascicule du brevet:
**12.02.92**

(51) Int. Cl.5: **C07H 21/04**, C12Q 1/68, C12Q 1/04

(21) Numéro de dépôt: **87400745.3**

(22) Date de dépôt: **03.04.87**

(54) **Sondes oligonucléotidiques et procédés pour la détection par hybridation des acides nucléiques de bactéries et autres êtres vivants.**

(30) Priorité: **04.04.86 FR 8604914**

(43) Date de publication de la demande:
**11.11.87 Bulletin 87/46**

(45) Mention de la délivrance du brevet:
**12.02.92 Bulletin 92/07**

(84) Etats contractants désignés:
**AT BE CH DE ES GB GR IT LI LU NL SE**

(56) Documents cités:
**EP-A- 0 155 359**
**EP-A- 0 174 226**
**WO-A-84/03716**

**PROC. NATL. ACAD. SCI. USA, vol. 82, octobre 1985, pages 6955-6959; D.J. LANE et al.: "Rapid determination of 16S ribosomal RNA sequences for phylogenetic analyses"**

**CHEMICAL ABSTRACTS, vol. 103, no. 5, 5 août 1985, page 95, résumé no. 32819k, Columbus, Ohio, US; J. FRYDENBERG et al.: "The sequence of 16S rRNA from mycoplasma strain PG50", & DNA 1985, 4(2), 127-137**

**J.Bact, vol. 164(1), pp 230-236 (1985)**

**DNA, vol. 4(2), pp 127-137 (1985)**

(73) Titulaire: **INSTITUT PASTEUR**
**25-28, rue du Docteur Roux**
**F-75724 Paris Cédex 15(FR)**

Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Marlière, Philippe**
**201, rue Lecourbe**
**F-75015 Paris(FR)**
Inventeur: **Grimont, Patrick**
**207, rue de Vaugirard**
**F-75015 Paris(FR)**

(74) Mandataire: **Gutmann, Ernest et al**
**S.C. Ernest Gutmann - Yves Plasseraud 67, boulevard Haussmann**
**F-75008 Paris(FR)**

**Description**

Les médecins, vétérinaires, phytopathologistes, et d'une manière générale les biologistes doivent souvent déterminer si un prélèvement (humain, animal, végétal, aliment ou autre) contient des bactéries ou éventuellement d'autres organismes. Actuellement, les seuls moyens utilisables pour détecter la présence de microorganismes vivants sont la microscopie (optique ou électronique), la culture, la détection immunologique et la détection par sondes nucléiques spécifiques.

La détection immunologique et la détection par sondes nucléiques spécifiques ne s'appliquent actuellement qu'à des organismes particuliers, par exemple Legionella pneumophilia ou Salmonella. Un réactif permettant de détecter un ou plusieurs organismes est en général incapable de détector les autres espèces ou genres (ce principe de spécificité est la raison d'être de ces réactifs).

L'examen microscopique est nécessairement limité à une petite partie de l'échantillon et les débris cellulaires ou autres particules peuvent rendre impossible la visualisation d'un organisme vivant identifiable comme tel.

Le culture n'est possible que pour un certain nombre d'organismes (surtout bactériens) et sur certains milieux et dans des conditions propres à ces organismes (bactéries aérobies ou anaérobies, exigentes en tel ou tel facteur de croissance, etc.). Aucun milieu de culture ne peut à lui seul permettre la croissance de toutes les bactéries cultivables. Des bactéries peuvent être aujourd'hui inconnues et non détectables, parce qu'on ne sait pas comment les cultiver. Dans exemples abondent où la présence de bactéries n'a pu être prouvée qu'après une ou plusieurs années de recherche : dans la maladie des légionnaires (épidémie de Philadelphie), les bactéries responsables que les prélèvements humains se sont révélés contenir par la suite n'étaient pas visibles après coloration usuelle au microscope et ne pouvaient pas être cultivées sur les milieux bactériologiques connus jusqu'alors. De même, des bactéries infectant le xylème des plantes ou longtemps été ignorées. Elles devaient être découvertes à l'occasion d'observations délicates sous le microscope. Comme dans le cas précédent, ces bactéries n'étaient pas cultivables sur les milieux connus à l'époque. Il résulte des exemples qui précèdent que l'absence de détection d'une contamination bactérienne dans un milieu à l'étude par les moyens classiquement utilisés ne peut nécessairement conduire à la conclusion que ce milieu est dépourvu de toute contamination bactérienne.

L'invention a pour but de remédier aux insuffisances des techniques classiques de détection, en d'autres termes de fournir un procédé qui permettre, soit de confirmer le caractère stérile d'un milieu étudié, par exemple d'un prélèvement humain, soit en révélant l'existence d'une contamination par exemple bactérienne avec un degré de fiabilité inconnue à ce jour.

On appréciera aisément les grands services qu'un tel procédé peut rendre dès lors qu'il permettrait de déceler une contamination d'un prélèvement par bactéries ou autres cellules, avec même une possibilité de dénombrement. Une telle détection ne pourrait, par exemple, que stimuler efficacement la recherche d'une bactérie dans des cas d'infections (humaine, animale, végétale) d'étiologie inconnue.

L'invention découle de l'intérêt qu'ont porté les inventeurs à l'étude des ARNs ribosomaux. Parmi l'ensemble des constituants macromoléculaires des êtres vivants, les acides ribonucléiques (ARN) ribosomaux apparaissent a priori comme de fiables témoins d'une contamination biologique (présence d'êtres vivants). Ceux-ci forment une part substantielle du matériel cellulaire. Ils sont nécessaires au fonctionnement de toutes les cellules vivantes connues.

L'ARN de la petite sous-unité ribosomique (dit ARN 16S en référence à sa constante de sédimentation en ultra-centrifugation avec gradient de saccharose) est maintenant connue à l'échelle nucléotidique pour une vingtaine d'organismes (des exemples de séquences nucléotidiques complètes sont donnés par Weisburg et coll. 1985, J. Bacteriol. vol. 164, n° 1, p. 230-236 ; Frydenberg et Christiansen, 1985, DNA vol. 4, n° 2, p. 127-137). Les nucléotides sont conventionnellement numérotés de l'extrémité 5′ de la molécule d'ARN 16S à l'extrémité 3′. L'ARN 16S de la bactérie Escherichia coli comprend 1542 nucléotides. Les molécules correspondantes chez les autres organismes vivants peuvent différer de celle d'E. coli dans leur nombre total de nucléotides et dans leur séquence nucléotidique.

C'est de la comparaison des séquences nucléotidiques des ARNs ribosomaux connus et la découverte de parties qui leur sont communes avec un degré d'identité, en tous les cas d'extrême similitude allant au-delà des niveaux de conservation de séquences auxquels ont pouvait s'attendre, qui ont conduit à l'invention. Il a ainsi été constaté que les variations entre les différents organismes ne sont pas réparties tout le long des séquences d'ARN 16S, mais se concentrent en certains endroits (surtout dans les hélices de la structure), tandis que certaines régions (les boucles en particulier) apparaissent beaucoup plus conservées. D'autre part, ces variations s'accordent de manière cohérente avec les grandes divisions taxonomiques. On peut ainsi définir dans les séquences d' ARN 16S des motifs universels, des motifs spécifiquement eucaryotes, bactériens (procaryotes), et des motifs propres à des groupes bactériens plus restreints.

Les ARNs 16S se sont ainsi révélés présenter une région universelle (c'est-à-dire commune aux Eucaryotes et aux Procaryotes) s'étendant du nucléotide 1492 au nucléotide 1506 (suivant la numérotation en vigueur pour la séquence de l'ARN 16S de E. coli) et une région spécifique des bactéries (eubactéries, archébactéries méthanogènes et halophiles) s'étendant du nucléotide 1527 au nucléotide 1541. On peut à cet égard se reporter au tableau sur la page suivante, dans lequel A, T, C, G désignent les 4 nucléotides de base entrant dans la constitution des ADNs ; il est entendu que le nucléotide désigné par un nombre de numérotation donné correspond à celui qui est placé sous le premier chiffre du nombre. Par exemple, le nombre 1490 correspond à l'uracyle (U) dans la séquence nucléotidique correspondant à l'ARN.

Les ARNs de transfert d'un grand nombre de bactéries se sont également révélés comporter des séquences communes, notamment la suivante :

UCAUAACCCGAAGGUC.

Cette séquence paraît cependant moins universelle que la région spécifique des bactéries mentionnées plus haut, qui s'étend du nucléotide 1527 au nucléotide 1541.

Les archébactéries se sont également révélées posséder une séquence commune à l'ensemble de ce genre bactérien, séquence qui présente la structure suivante :

UUCGACGAGRUGGGGCGC

dans laquelle R est A ou G.

L'invention découle donc des constatations qui précèdent, et elle fournit des séquences nucléotidiques spécifiques complémentaires des précédentes, lesquelles constituent autant de sondes, applicables, pour certaines d'entres elles, de façon universelle à la détection d'une contamination cellulaire dans tout milieu, pour d'autres, au classement des cellules éventuellement détectées parmi les catégories suivantes :

ARNs

Sonde eubactérienne

Sonde universelle

```
d--TTCAGCATTGTTCCA 5'              d-GACCTAGTGGAGGAA 5'
    1490'   1500'   1510'   1520'    1530'      1542
EUBACTERIES
E coli      ...GUGAAGUCGUAACAAGGUAACCGUAGGGGAACCUGCGGUUGGAUCACCUCCUUA
B fragilis  ...GCUAAGUCGUAACAAGGUAGCCGUACUGGAAGGUGCGGCUGGAACACCUCCUUUCU
B subtilis  ...GUGAAGUCGUAACAAGGUAGCCGUAUCGGAAGGUGCGGCUGGAUCACCUCCUUUCUA
mycoplasme  ...GUGAAGUCGUAACAAGGUAUCCGUACGGGAACGUGCGGAUGGAUCACCUCCUUU
An nidulans ...GUGAAGUCGUAACAAGGUAGCCGUACCGGAAGGUGCGGCUGGAUCACCUCCUUU

CHLOROPLASTES
maïs        ...GUGAAGUCGUAACAAGGUAGCCGUACUGGAAGGUGCGGCUGGAUCACCUCCUUU
tabac (...) ...GUGAAGUCGUAACAAGGUAGCCGUACUGGAAGGUGCGGCUGGAUCACCUCCU

ARCHAEBACTERIES
M vaniellii       ...GUGAAGUCGUAACAAGGUAGCCGUAGGGGAACCUGCGGCUGGAUCACCUCC
H volcanii        ...CUUAAGUCGUAACAAGGUAGCCGUAGGGGAAUCUGCGGCUGGAUCACCUCCUU
S acidocaldarius  ...GAGAAGUCGUAACAAGGUAGCCGUAGGGGAACCUGCGGCUGGAUCACCUCAUAUAUUUA...

EUCARYOTES
D discoideum ...GAGAAGUCGUAACAAGGUAUCCGUAGGUGAACCUGCGGAAGGAUCAUUU
S cerevisiae ...UAAAAGUCGUAACAAGGUUUCCGUAGGUGAACCUGCGGAAGGAUCAUUA
vertébrés    ...UAAAAGUCGUAACAAGGUUUCCGUAGGUGAACCUGCGGAAGGAUCAUUA

MITOCHONDRIES
As nidulans ...GUUAAGUCGAAAUAUGGUUCGUGUUAAUGGAAAUUGCACGGGAUGAAUUA
homme       ...GUAAGUGUACUGGAAAGUGCACUUGGACGAAC...
```

- en bactéries, y inclues certaines archaebactéries, plus particulièrement les archaebactéries méthanogènes et halophiles
- archaebactéries

et dans toutes autres catégories d'organismes, dans le cas de réponse positive dans des essais d'hybridation avec la "sonde universelle" et de réponse négative avec les sondes autorisant le classement des micro-organismes détectés dans les catégories précédemment désignées.

Les séquences du genre en question sont aisément accessibles par synthèse nucléotidique. L'invention concerne donc plus particulièrement un pentadécadésoxyribonucléotide (séquence d'acide désoxyribonucléique de 15 nucléotides ou en abrégé, 15-mère) complémentaire de la région 1492-1506 des ARNs 16S, ce 15-mère étant capable de s'hybrider avec le fragment homologue contenu dans l'ARN 16S de tous les êtres vivants ainsi qu'avec la partie de l'acide désoxyribonucléique (ADN) qui code pour cette partie d'ARN et qui est contenue dans le génome de tous les êtres vivants.

Elle concerne aussi
- les sondes que constituent les oligonucléotides complémentaires de la séquence UCAUAACCCGAAGGUC déjà nommée pour la détection de micro-organismes susceptibles d'être classés parmi les bactéries et
- les sondes complémentaires de la séquence UUCGACGAGRUGGGGCGC susmentionnée, ces sondes étant elles-mêmes caractéristiques des archaebactéries.

L'invention concerne enfin une séquence d'ADN de 15 nucléotides (15-mère) complémentaire de la région 1527-1541 des ARNs 16S, ce 15-mère étant capable de s'hybrider avec le fragment homologue contenu dans l'ARN 16S des bactéries et autres procaryotes ainsi qu'avec les gènes correspondants dans l'ADN de ces organismes, mais pas ou très peu avec l'ARN correspondant des cellules eucaryotes (levures, champignons, protozoaires, tissus humains, animaux et végétaux) ou les gènes correspondants dans l'ADN de ces organismes.

L'invention concerne par conséquent plus particulièrement les compositions suivantes :

1°/ Sonde ci-après dite "Sonde universelle" :

La sonde réalisée par synthèse oligonucléotidique a la séquence suivante :
(5')d - ACC TTG TTA CGA CTT (3')
où (5') et (3') indiquent les extrémités 5' et 3' du polymère (et donc sa polarité) et où les lettres indiquent les désoxynucléotides suivants : A, résidu adénylique ; C, résidu cytidylique ; T, résidu thymidylique ; et G, résidu guanidylique ; les espaces ne servant qu'à la lisibilité de la séquence qui contient 15 nucléotides.

Cette sonde est parfaitement complémentaire de la portion 1492-1506 (selon la numérotation en vigueur pour Escherichia coli) de l'ARN 16S de ce micro-organisme et de portions correspondantes des ARNs publiés de toutes autres bactéries, cellules procaryotes et eucaryotes, et, bien qu'à un degré moindre avec des ARNs de mitochondries. Le spécialiste en déduit la probabilité quasi-absolue que cette sonde doit s'apparier aussi à des ARNs de toutes cellules existant dans la nature. Cette sonde est aussi capable de s'apparier avec la portion homologue de l'ADN des gènes qui codent pour ces ARNs 16S et ARNs eucaryotiques correspondants.

L'invention concerne naturellement aussi la sonde complémentaire à la "sonde universelle" susdite et ayant la séquence nucléotidique suivante :
(5')d - AAG TCG TAA CAA GGT (3')
capable de s'apparier avec la même portion d'ADN génomique (gènes codant pour les ARNs 16S et ARN correspondants), en raison de sa nature bicaténaire, même si elle n'est plus capable de s'apparier avec l'ARN 16S (qui ne comporte qu'une seule chaîne).

2°/ Sonde ci-après dite "Composition spécifique des eubactéries et de certaines archaebactéries" ou "sonde eubactérienne"

La sonde préférée réalisée par synthèse oligonucléotidique a la séquence suivante :
(5')d - AAG GAG GTG ATC CAG (3')
où (5'), (3'), A, C, T et G ont les significations sus-indiquées.

Cette séquence correspond au complément du consensus de la portion 1527-1541 (selon la numérotation en vigueur pour Escherichia Coli) des ARNs 16S des bactéries et plus précisément, des eubactéries. Cette séquence n'est pas parfaitement complémentaire de toutes les séquences d'ARN 16S publiées à ce jour pour les bactéries, mais elle n'en diffère au plus que par un ou deux nucléotides. Par contre, la séquence de cette composition ne peut s'apparier que faiblement à la région homologue des ARNs 18S des eucaryotes et 12S des mitochondries (ces ARNs correspondent aux ARNs 16S des bactéries).

L'invention concerne naturellement aussi toute sonde susceptible de s'apparier avec les mêmes régions des ARNs et/ou ADN génomique, en particulier une séquence décalée d'un nucléotide vis-à-vis de la "sonde eubactérienne" sus-indiquée, en ce qu'elle s'apparie avec la séquence UGGAUCACCUCCUUA (la sonde étant alors constituée par la séquence :

(3′) ACCTAGTGGAGGAAA (5′) ou (si on inverse le sens de lecture)

(5′)d - AAAGGAGGTGATCCA (3′)

que l'on retrouve dans la plupart des bactéries.

Elle concerne naturellement également toute sonde d'oligonucléotides plus petite, dont la séquence est néanmoins contenue dans la susdite "sonde spécifique des eubactéries" (ou dans la sonde contenant la "séquence décalée").

Cette sonde contient néanmoins au moins la séquence TGGAGGAA (5′) (complémentaire de la région de l'ARN 16S s'étendant entre les nucléotides 1534 et 1541), notamment

(5′)d - AAGGAGGT (3′)

(5′)d - AAAGGAGGT (3′)

(5′)d - AAGGAGGTG (3′)

(5′)d - AAGGAGGTGA (3′)

(5′)d - AAGGAGGTGAT (3′)

(5′)d - AAGGAGGTGATC (3′)

(5′)d - AAGGAGGTGATCC (3′)

(5′)d - AAGGAGGTGATCCA (3′).

Les séquences complémentaires des précédentes font également partie du groupe de sondes conformes à l'invention.

Il convient néanmoins de souligner le fait que les sondes les plus longues sont préférées en raison de leur plus grande stabilité d'appariement. Par ailleurs, la substitution d'un nucléotide par un autre au sein d'une même séquence les rendrait peut-être plus affines pour les ARNs 16S d'un groupe bactérien déterminé, mais affaiblirait leur complémentarité vis-à-vis de la majorité des autres. D'autre part, l'allongement s'effectuant en 3′ par tout ou partie du motif CCGCA ne ferait pas gagner de spécificité, le complément de son motif se trouvant représenté dans l'ARN 18S des vertébrés.

3°/ Sonde spécifique des archarbactéries

L'invention concerne enfin plus particulièrement une sonde spécifique des archaebactéries, cette sonde présentant une séquence d'au moins 8 nucléotides contenus dans la séquence suivante :

AAGCTGCTCR′ACCCCGCG

et au plus les 18 nucléotides de la séquence, dans laquelle R′ et T ou C.

## CONDITIONS GENERALES D'UTILISATION DES SONDES

Les sondes universelles et les sondes plus spécifiques des eubactéries, d'une part, et des archaebactéries, d'autre part (et les compositions complémentaires) sont avantageusement marquées. Tout marqueur classiquement envisagé peut être utilisé. Les compositions peuvent être marquées à l'aide de traceurs radio-actifs comme $^{32}$P, $^{35}$S, $^{125}$I, $^{3}$H, $^{14}$C pour ne citer que les traceurs les plus communément utilisés. Le marquage radioactif peut être réalisé selon une méthode quelconque comme par exemple le marquage terminal en 3′ ou 5′ en utilisant un nucléotide radiomarqué, la polynucléotide kinase (avec ou sans déphosphorylation par une phosphatase) ou une ligase (selon l'extrémité à marquer). Dans ces cas, la composition marquée aurait 16 nucléotides (le nucléotide radioactif ajouté en 3′ ou 5′ étant quelconque. Une des sondes de l'invention (y compris une composition complémentaire) peut servir de matrice pour la synthèse d'une courte chaîne (d'environ 15 nucléotides) constituée de nucléotides radioactifs ou d'un ensemble de nucléotides radioactifs et non-radioactifs. Les sondes de l'invention peuvent aussi être réalisées par une synthèse chimique utilisant un ou plusieurs nucléotides radioactifs. Une autre méthode de marquage radioactif est l'iodation chimique des sondes de l'invention aboutissant à la fixation sur les oligomères de plusieurs atomes de $^{125}$I. Si l'une des sondes de l'invention (ou d'une sonde complémentaire) est rendue radioactive pour être utilisée en hybridation avec un ARN ou un ADN non radioactifs, la méthode de détection de l'hybridation dépendra du tracteur radioactif utilisé et pourra s'appuyer sur l'autoradiographie, la scintillation liquide, le comptage gamma ou toute autre technique permettant de détecter l'émission du rayonnement ionisant émis par le traceur radioactif.

Un marquage non radioactif peut être aussi utilisé, associant aux sondes de l'invention des résidus ayant des propriétés immunologiques (antigènes, haptènes), une affinité spécifique pour certains réactifs (ligands), des propriétés permettant la complétion de réactions enzymatiques détectables (enzymes ou coenzymes, substrat d'enzyme, ou autre substance intervenant dans une réaction enzymatique), des propriétés physiques propres comme la fluorescence ou l'émission ou l'absorption de lumière à une longueur d'onde quelconque, pour ne citer que des exemples. Des anticorps reconnaissant spécifiquement les hybrides ADN-ARN peuvent aussi être utilisés.

Le marquage chimique qui vient d'être évoqué peut être réalisé lors de la synthèse chimique des sondes de l'invention, les résidus adénosine, guanosine, cytidine et thymidine pouvant être couplés à d'autres résidus chimiques permettant la détection de la composition ou les hybrides formés entre la composition et un fragment d'ADN ou d'ARN complémentaire. Cependant, la séquence de la composition utilisant des nucléotides modifiés par couplage à d'autres résidus chimiques serait la même que la séquence nucléotidique d'une des sondes de l'invention.

L'invention concerne aussi les procédés de détection par hybridation eux-mêmes mettant en oeuvre des sondes conformes à l'invention, celles-ci ayant au préalable été marquées ou rendues aptes à être détectées comme il a été dit plus haut.

Le choix de l'une ou de l'autre des sondes de l'invention dépendra du but recherché : détection de cellules procaryotes et eucaryotes (composition universelle), détection ou localisation d'ADN ou de fragments d'ADN portant des gènes codant pour les ARN de la série 16S et provenant indifféremment de cellules procaryotes ou eucaryotes (composition universelle ou sonde qui lui est complémentaire), détection exclusive de cellules bactériennes (sonde spécifique des eubactéries), détection ou localisation d'ARN ou de fragments d'ADN portant les gènes codant pour les ARN 16S bactériens (sonde spécifique des eubactéries ou sone qui lui est complémentaire).

L'utilisation d'une ou plusieurs sondes de l'invention ne peut être limitée à une technique particulière d'hybridation.

S'il s'agit de détecter des cellules provenant de (ou étant elles-mêmes des) organismes vivants, les ARN et/ou l'ADN de ces cellules doivent être rendus accessibles (par lyse partielle ou totale des cellules en utilisant des procédés chimiques ou physiques) et mis en contact avec une ou plusieurs des sondes de l'invention, elle(s)-même(s) rendue(s) détectable(s). Ce contact peut se faire sur un support approprié tel que filtre de nitro-cellulose, de cellulose (modifiée ou non), de nylon (pour ne citer que les supports usuels) ou sur coupe histologique ou frottis de cellules ou, sans support, en milieu liquide. Ce contact a lieu dans des conditions optimales ou restrictives (c'est-à-dire ne permettant la formation d'hybrides que si les séquences sont parfaitement homologues sur une longueur de molécule d'autant plus importante que les conditions sont plus "restrictives") de température, de concentration ionique, avec ou sans substances abaissant la térature optimale d'appariement des acides nucléiques (formamide, diméthylsulfoxyde, urée), avec ou sans substances diminuant apparemment le volume réactionnel (dextran, sulfate de dextran).

L'élimination des molécules ou fragments de molécules de la sonde n'ayant pas réagi sur un support peut être réalisée par lavage avec une solution tampon de force ionique appropriée et à une température appropriée, avec ou sans traitement par la nucléase S1 ou autre enryme digérant l'ADN ou l'ARN monocaténaires, mais ne digérant pas les hybrides ADN-ARN ou l'ADN bicaténaire. En milieu liquide, les molécules (ou fragments) de la sonde s'étant appariés avec des fragments d'ARN ou d'ADN peuvent être séparés de ceux qui n'ont réagit par chromatographie sur hydroxyapatite ou, après traitement à la nucléase S1, par toutes méthodes séparant les fragments bicaténaires des nucléotides libres (chromatographie sur hydroxyapatite, sur DEAE-cellulose, ou d'exclusion ; précipitation sélective ; dialyse ; pour ne citer que les moyens les plus usuels).

Ensuite, les molécules (ou fragments) de la composition utilisée sont détectées par la méthode la plus avantageuse dépendant du type de marquage choisi.

Les détails techniques décrits dans diverses publications (Maniatis et al. 1982, "Molecular cloning, A laboratory manual", Cold Spring Harbor Laboratory ; Grimont et al. 1985, J. Clin. Microbiol. vol. 21, n° 3, pp. 431-437) ou brevets (français : 84 12250, 78 10975, 81 24631 ; européens : 0133288, 0063879) pourront avantageusement être appliqués avec les compositions de l'invention.

S'il s'agit de localiser les fragments d'ADN chromosomiques portant les gènes codant pour les ARN 16S (ou ARN correspondants) après traitement de l'ADN par une ou plusieurs enzymes de restriction, séparation des fragments par électrophorèse ou chromatographie, et dénaturation des fragments d'ADN (c'est-à-dire séparation des deux chaînes), une des sondes de l'invention est mise en contact avec les fragments d'ADN dans des conditions favorisant l'hybridation et, après le temps nécessaire à la complétion de l'hybridation, les fragments non hybridés de la composition seront séparés des fragments hybridés et le marquage révélé comme il a été dit pour la détection des cellules.

Les détails techniques décrits dans le brevet européen 0120658 (John A. Webster Jr) ou dans l'ouvrage de Maniatis et al. ou dans l'article de Grimont et al. (cités plus haut) peuvent être appliqués avec l'une ou l'autre des sondes de l'invention. Notons que, pour la localisation des fragments d'ADN chromosomiques portant les gènes codant pour les ARN 16S, une des sondes de l'invention et une sonde qui lui serait exactment complémentaire donnerait exactement les mêmes résultats.

D'une façon générale, les différentes sondes de l'invention peuvent également être contenues dans des ADNs recombinants permettant leur clonage, dès lors que la présence d'un ADN hétérologue ne nuirait pas à la spécificité des sondes dans les applications envisagées.

Enfin, il est entendu que font également partie de l'invention les sondes dans lesquelles les groupes thymidine sont remplacés par des groupes uracyle (ces sondes devant donc être considérées comme strictement équivalentes aux sondes plus particulièrement revendiquées) même si leur synthèse est plus difficile à effectuer.

**Revendications**

1. Sonde pour détecter la présence de bactéries dans un échantillon ou pour localiser parmi des fragments d'ADN séparés par électrophorèse ou chromatographie ceux qui portent les gènes codant pour les ARNs 16S des bactéties, caractérisée en ce qu'elle est constituée, soit d'un pentadécadésoxy-ribonucléotide de séquence

$(5')d - AAG\ GAG\ GTG\ ATC\ CAG\ (3')$

où $(5')$ et $(3')$ indiquent les extrémités $5'$ et $3'$ du polymère et où les lettres indiquent les résidus (nucléotides) suivants : A, résidu adénylique ; C, résidu cytidylique ; T, résidu thymidylique ; et G, résidu guanidylique ; soit d'un pentadécadesoxyribonucléotide présentant la séquence complémentaire

$(5')d - CTG\ GAT\ CAC\ CTC\ CTT\ (3')$.

2. Sonde pour détecter la présence de bactéries dans un échantillon ou pour localiser parmi des fragments d'ADN séparés par électrophorèse ou chromatographie ceux qui portent les gènes codant pour les ARNs 16S des bactéries, caractérisée en ce qu'elle est constituée par une séquence

$(3')\ ACCTAGTGGAGGAAA\ (5')$

ou par la séquence complémentaire correspondante.

3. Sonde pour détecter la présence de bactéries dans un échantillon ou pour localiser parmi des fragments d'ADN séparés par électrophorèse ou chromatographie ceux qui portent les gènes codant pour les ARNs 16S bactéries, caractérisée en ce qu'elle est constituée par une séquence sélectionnée dans le groupe des séquences suivantes

$(5')d - AAGGAGGT\ (3')$

$(5')d - AAAGGAGGT\ (3')$

$(5')d - AAGGAGGTG\ (3')$

$(5')d - AAGGAGGTGA\ (3')$

$(5')d - AAGGAGGTGAT\ (3')$

$(5')d - AAGGAGGTGATC\ (3')$

$(5')d - AAGGAGGTGATCC\ (3')$

$(5')d - AAGGAGGTGATCCA\ (3')$

ou parmi les séquences complémentaires correspondantes.

4. Sonde pour détecter les cellules d'organismes eucaryotes ou procaryotes (y compris les bactéries) dans un échantillon ou pour localiser parmi des fragments d'ADN séparés par électrophorèse ou chromatographie ceux qui portent les gènes codant pour les ARNs de la série 16S de ces cellules, caractérisée en ce qu'elle est constituée, soit d'un pentadécadésoxyribonucléotide de séquence

$(5')d - ACC\ TTG\ TTA\ CGA\ CTT\ (3')$

où $(5')$ et $(3')$ indiquent les extrémités $5'$ et $3'$ du polymère et où les lettres indiquent les résidus (nucléotides) suivants : A, résidu adénylique ; C, résidu cytidylique ; T, résidu thymidylique ; et G, résidu guanidylique, soit d'un pentadécadésoxyribonucléotide présentant la séquence complémentaire

$(5')d - AAG\ TCG\ TAA\ CAA\ GGT\ (3')$.

5. Sonde pour détecter la présence d'archaebactéries dans un échantillon ou pour localiser parmi des fragments d'ADN séparés par électrophorèse ou chromatographie ceux qui portent les gènes codant pour les ARNs 16S des archaebactéries, caractérisée en ce qu'elle est constituée par une séquence d'au moins 8 nucléotides contenus dans la séquence suivante

$AAGCTGCTCR'ACCCCGCG$

et au plus les 18 nucléotides de la séquence, dans laquelle R' est T ou C ou par une séquence complémentaire de la précédente.

6. Sonde selon l'une quelconque des revendications 1 à 5, caractérisée en ce que la sonde oligonucléotidique ayant l'une des susdites séquences est marquée radioactivement, (notamment par un ou deux nucléotides radioactifs de nature quelconque à l'une ou l'autre des extrémités ou aux deux) ou par des marqueurs, notamment enzymatiques, ligands, luminescents ou fluorescents.

7. Procédé pour détecter les bactéries dans un échantillon, caractérisé en ce que, après avoir rendu les acides nucléiques de ces bactéries accessibles et monocaténaires, on les met en contact avec une sonde selon l'une quelconque des revendications 1, 2 et 3 dans des conditions favorables à l'hybridation et on détecte les hybrides éventuellement formés.

8. Procédé pour détecter les cellules d'organismes eucaryotes ou procaryotes (y compris les bactéries) dans un échantillon, caractérisé en ce que, après avoir rendu les acides nucléiques de ces cellules accessibles et monocaténaires, on les met en contact avec une sonde selon la revendication 4 dans des conditions favorables à l'hybridation et on détecte les hybrides éventuellement formés.

9. Procédé pour détecter les archaebactéries dans un échantillon caractérisé en ce que, après avoir rendu les acides nucléiques de ces archaebactéries accessibles et monocaténaires, on les met en contact avec une sonde selon la revendication 5, dans des conditions favorables à l'hybridation et on détecte les hybrides éventuellement formés.

10. Procédé pour localiser parmi des fragments d'ADN séparés par électrophorèse ou chromatographie ceux qui portent des gènes codant pour les ARNs de la série 16S, caractérisé par la mise en contact de ces fragments d'ADN séparés et dénaturés avec une composition selon l'une quelconque des revendications 1 à 5, dans des conditions autorisant une hybridation éventuelle et par la détection des hybrides éventuellement formés.

## Claims

1. Probe for detecting the presence of bacteria in a sample or for localizing among DNA fragments separated by electrophoresis or chromatography those which carry the genes which code for the 16S RNAs of bacteria, characterized in that it consists either of a pentadecadeoxyribonucleotide of sequence
   (5')d - AAG GAG GTG ATC CAG (3')
   where (5') and (3') indicate the 5' and 3' ends of the polymer and where the letters indicate the following residues (nucleotides): A, adenyl residue; C, cytidyl residue; T, thymidyl residue; and G, guanidyl residue; or of a pentadecadeoxyribonucleotide possessing the complementary sequence
   (5')d - CTG GAT CAC CTC CTT (3').

2. Probe for detecting the presence of bacteria in a sample or for localizing among DNA fragments separated by electrophoresis or chromatography those which carry the genes which code for the 16S RNAs of bacteria, characterized in that it consists of a sequence
   (3') ACCTAGTGGAGGAAA (5')
   or of the corresponding complementary sequence.

3. Probe for detecting the presence of bacteria in a sample or for localizing among DNA fragments separated by electrophoresis or chromatography those which carry the genes which code for the 16S RNAs of bacteria, characterized in that it consists of a sequence selected from the following group of sequences:
   (5')d - AAGGAGGT (3')
   (5')d - AAAGGAGGT (3')
   (5')d - AAGGAGGTG (3')
   (5')d - AAGGAGGTGA (3')
   (5')d - AAGGAGGTGAT (3')
   (5')d - AAGGAGGTGATC (3')
   (5')d - AAGGAGGTGATCC (3')
   (5')d - AAGGAGGTGATCCA (3')
   or from the corresponding complementary sequences.

4. Probe for detecting the cells of eucaryotic or procaryotic organisms (including bacteria) in a sample or for localizing among DNA fragments separated by electrophoresis or chromatography those which carry the genes which code for the RNAs of the 16S series of these cells, characterized in that it consists either of a pentadecadeoxyribonucleotide of sequence

(5')d - ACC TTG TTA CGA CTT (3')

where (5') and (3') indicate the 5' and 3' ends of the polymer and where the letters indicate the following residues (nucleotides) : A, adenyl residue; C, cytidyl residue; D, thymidyl residue; and G, guanidyl residue; or a pentadecadeoxyribonucleotide possessing the complementary sequence

(5')d - AAG TCG TAA CAA GGT (3').

5. Probe for detecting the presence of archaebacteria in a sample or for localizing among DNA fragments separated by electrophoresis or chromatography those which carry the genes which code for the 16S RNAs of archaebacteria, characterized in that it consists of a sequence of at least 8 nucleotides contained in the following sequence:

AAGCTGCTCR'ACCCCGCG

and at most the 18 nucleotides of the sequence, in which R' is T or C, or of a sequence complementary to the above.

6. Probe according to any one of Claims 1 to 5, characterized in that the oligonucleotide probe having one of the abovementioned sequences is radioactively labelled (in particular, with one or two radioactive nucleotides of any nature at either or both of the ends) or labelled, in particular; with enzyme, ligand, luminescent or fluorescent labels.

7. Method for detecting the bacteria in a sample, characterized in that, after the nucleic acids of these bacteria have been made accessible and single-stranded, they are brought into contact with a probe according to any one of Claims 1, 2 and 3 under conditions which favour hybridization, and any hybrids which may be formed are detected.

8. Method for detecting the cells of eucaryotic or procaryotic organisms (including bacteria) in a sample, characterized in that, after the nucleic acids of these cells have been made accessible and single-stranded, they are brought into contact with a probe according to Claim 4 under conditions which favour hybridization, and any hybrids wich may be formed are detected.

9. Method for detecting archaebacteria in a sample, characterized in that, after the nucleic acids of these archaebacteria have been made accessible and single-stranded, they are brought into contact with a probe according to Claim 5 under conditions which favour hybridization, and any hybrids which may be formed are detected.

10. Method for localizing among DNA fragments separated by electrophoresis or chromatography those which carry genes which code for the RNAs of the 16S series, characterized by bringing these separated and denatured DNA fragments into contact with a composition according to any one of Claims 1 to 5, under conditions which permit possible hybridization, and by the detection of any hybrids which may be formed.

**Patentansprüche**

1. Sonde zum Nachweisen von Bakterien in einer Probe oder zum Auffinden derjenigen DNA-Fragmente aus elektrophoretisch oder chromatographisch aufgetrennten DNA-Fragmenten, die die Gene tragen, die die 16S-RNAs der Bakterien codieren, dadurch gekennzeichnet, daß sie entweder aus einem Pentadecadesoxyribonucleotid der Sequenz

(5')d - AAG GAG GTG ATC CAG (3')

besteht, in der (5') und (3') das 5'- und 3'-Ende des Polymers und die Buchstaben die folgenden Reste (Nucleotide) bedeuten: A Adenylsäurerest, C Cytidylsäurerest, T Thymidylsäurerest, und G Guanidylsäurerest, oder aus einem Pentadecadesoxyribonucleotid, das die Komplementärsequenz

(5')d - CTG GAT CAC CTC CTT (3')

darstellt.

2. Sonde zum Nachweisen von Bakterien in einer Probe oder zum Auffinden derjenigen DNA-Fragmente aus elektrophoretisch oder chromatographisch aufgetrennten DNA-Fragmenten, die die Gene tragen, die die 16S-RNAs der Bakterien codieren, dadurch gekennzeichnet, daß sie aus einer Sequenz
(3') ACCTAGTGGAGGAAA (5')
oder der entsprechenden Komplementärsequenz besteht.

3. Sonde zum Nachweisen von Bakterien in einer Probe oder zum Auffinden derjenigen DNA-Fragmente aus elektrophoretisch oder chromatographisch aufgetrennten DNA-Fragmenten, die die Gene tragen, die die 16S-RNAs der Bakterien codieren, dadurch gekennzeichnet, daß sie aus einer der folgenden Sequenzen
(5')d - AAGGAGGT (3')
(5')d - AAAGGAGGT (3')
(5')d - AAGGAGGTG (3')
(5')d - AAGGAGGTGA (3')
(5')d - AAGGAGGTGAT (3')
(5')d - AAGGAGGTGATC (3')
(5')d - AAGGAGGTGATCC (3')
(5')d - AAGGAGGTGATCCA (3')
oder aus einer der entsprechenden Komplementärsequenzen besteht.

4. Sonde zum Nachweisen der Zellen aus eukaryontischen oder prokaryontischen Organismen (einschließlich der Bakterien) in einer Probe oder zum Auffinden derjenigen DNA-Fragmente aus elektrophoretisch oder chromatographisch aufgetrennten DNA-Fragmenten, die die Gene tragen, die die 16S-RNAs dieser Zellen codieren, dadurch gekennzeichnet, daß sie entweder aus einem Pentadecadesoxyribonucleotid der Sequenz
(5')d - ACC TTG TTA CGA CTT (3')
besteht, in der (5') und (3') das 5'- und 3'-Ende des Polymers und die Buchstaben die folgenden Reste (Nucleotide) bedeuten: A Adenylsäurerest, C Cytidylsäurerest, T Thymidiylsäurerest, und G Guanidylsäurerest, oder aus einem Pentadecadesoxyribonucleotid, das die Komplementärsequenz
(5')d - AAG TCG TAA CAA GGT (3')
darstellt.

5. Sonde zum Nachweisen von Archaebakterien in einer Probe oder zum Auffinden derjenigen DNA-Fragmente aus elektrophoretisch oder chromatographisch aufgetrennten DNA-Fragmenten, die die Gene tragen, die die 16S-RNAs der Archaebakterien codieren, dadurch gekennzeichnet, daß sie aus einer Sequenz aus mindestens 8 der in der folgenden Sequenz
AAGCTGCTCR'ACCCCGCG
enthaltenen Nucleotide und höchstens aus den 18 Nucleotiden dieser Sequenz, in der R' T oder C bedeutet, oder aus einer Komplementärsequenz zu der obengenannten besteht.

6. Sonde nach einem der Ansprüche 1 bis 5, dadurch gekennzeichnet, daß die Oligonucleotidsonde mit einer der obenerwähnten Sequenzen radioaktiv markiert ist, insbesondere mit einem oder zwei beliebigen radioaktiven Nukleotiden entweder an einem der beiden Enden oder an beiden Enden, oder mit Markern, insbesondere enzymatischen, Liganden-, lumineszierenden oder fluoreszierenden.

7. Verfahren zum Nachweisen der Bakterien in einer Probe, dadurch gekennzeichnet, daß, nachdem die Nucleinsäuren der Bakterien verfügbar und einzelsträngig gemacht wurden, sie mit einer Sonde nach einem der Ansprüche 1, 2 und 3 unter günstigen Hybridisierungsbedingungen in Kontakt gebracht werden und die gegebenenfalls gebildeten Hybride nachgewiesen werden.

8. Verfahren zum Nachweisen der Zellen aus eukaryontischen oder prokaryontischen Organismen (einschließlich der Bakterien) in einer Probe, dadurch gekennzeichnet, daß, nachdem die Nucleinsäuren der Zellen verfügbar und einzelsträngig gemacht wurden, sie mit einer Sonde nach Anspruch 4 unter günstigen Hybridisierungsbedingungen in Kontakt gebracht werden, und die gegebenenfalls gebildeten Hybride nachgewiesen werden.

9. Verfahren zum Nachweisen der Archaebakterien in einer Probe, dadurch gekennzeichnet, daß, nachdem die Nucleinsäuren der Archaebakterien verfügbar und einzelsträngig gemacht wurden, sie mit einer Sonde nach Anspruch 5 unter günstigen Hybridisierungsbedingungen in Kontakt gebracht werden, und die gegebenenfalls gebildeten Hybride nachgewiesen werden.

10. Verfahren zum Auffinden derjenigen DNA-Fragmente aus elektrophoretisch oder chromatographisch aufgetrennten DNA-Fragmenten, die Gene tragen, die die 16S-RNAs codieren, gekennzeichnet durch das Inkontaktbringen dieser aufgetrennten und denaturierten DNA-Fragmente mit einer Zusammensetzung nach einem der Ansprüche 1 bis 5 unter Bedingungen, die eine mögliche Hybridisierung zulassen, und durch das Nachweisen von gegebenenfalls gebildeten Hybriden.